# EUROPEAN PATENT APPLICATION

(11) **EP 3 662 895 A1**
(43) Date of publication of application: **10.06.2020**
(21) Application number: 18211148.4
(22) Date of filing: 07.12.2018
(51) Int. Cl.: A61K 9/16, A61K 9/20, A61K 31/606

(54) **A PROCESS FOR MANUFACTURING REDUCING SUGAR-FREE 5-ASA TABLET CORES**

(71) Applicant: Tillotts Pharma AG, 4310 Rheinfelden (CH)
(72) Inventor: BRUNO, Cristina, 4310 Rheinfelden (CH); VARUM, Felipe, 4310 Rheinfelden (CH); BUSER, Thomas, CH-4412 Nuglar (CH); CETINKAYA-COSKUN, Yalcin, 4310 Rheinfelden (CH); BRAVO GONZÁLEZ, Roberto Carlos, 4310 Rheinfelden (CH)
(74) Representative: Beck Greener LLP

(57) **Abstract**

A process for manufacturing 5-aminosalicylic acid (5-ASA) tablet cores. The process comprises the steps of wet granulating a granulation composition comprising 5-ASA, a granulation liquid, and at least one binder using a high-shear granulator to form 5-ASA granules; drying the 5-ASA granules to form dry granules; blending the dry granules with at least one filler and optionally one or more additional pharmaceutical excipients to form a compression blend; and compressing the compression blend to form 5-ASA tablet cores. The granulation composition and the compression blend do not comprise a reducing sugar.

## Description

The present invention relates to a process for manufacturing 5-aminosalicylic acid (5-ASA, otherwise known as mesalazine or mesalamine) tablet cores for an oral drug formulation, and preferably for a modified release drug formulation for oral administration. In particular, it relates to a process for preparing 5-ASA granules using wet high-shear granulation, followed by compression into tablets in the absence of any reducing sugars.

5-ASA is an anti-inflammatory agent that has particular application in the local treatment of inflammatory bowel disease (IBD) (*e*.*g*. ulcerative colitis and Crohn's disease) and also in the prevention of colon cancer or colorectal cancer (primarily in colitis patients).

In such conditions, drug targeting to the colon would maximise the therapeutic effectiveness of the treatment. Various formulations have been developed for the colonic delivery of drugs such as 5-ASA (see for example, WO2007/122374, WO2013/164315, WO2015/062640 and WO2013/164316). These formulations typically comprise a core comprising the drug and a coating for the core. The coating typically comprises an enteric material which is designed to allow the release of the drug in the more alkaline environment of the colon. The high bacterial population of the colon has also been exploited in developing colonic drug delivery dosage forms through the use, as digestible carrier materials, of naturally occurring complex polysaccharides that constitute substrates for the numerous enzymes produced by the resident colonic bacteria.

Various processes are known for the preparation of 5-ASA tablet cores, usually involving granulation of 5-ASA with one or more excipients as a first step. During granulation (or agglomeration), dry primary powder particles are processed to adhere to form granules (or agglomerates). Granulation of powders is performed for many reasons. For example, granulation can prevent segregation of components in a powder mixture by reducing the variation in size and/or density of the particles in the mixture and improving homogeneity. It can also improve the flow properties (flowability) and compaction characteristics (compactibility) of a powder mixture.

5-ASA powder typically has poor flowability, low density and poor compactibility. 5-ASA densification and/or agglomeration prior to compression into tablets is therefore required to ensure consistent tablet manufacturing (*e*.*g*. uniform 5-ASA content, and consistent tablet hardness and weight).

Several industrial processes are known for densifying 5-ASA powder including dry and wet granulation processes. For example, WO 98/26767 and WO 2009/047802 disclose the wet granulation of mesalazine and a polyvinylpyrolidone (PVP) binder.

Wet granulation is typically performed by adding and mixing a granulation liquid into a dry powder mixture to promote the formation of bonds between primary particles. Mixing can be performed using a mechanical mixer (*e*.*g*. stirrers or paddles) or by introducing an air stream into the mixture known as fluid bed granulation or agglomeration. However, in order to obtain 5-ASA granules having acceptable density when using low-shear processes such as fluid bed granulation, powder pre-compaction is normally required, making the process more complex, variable and less economical.

High-shear wet granulation of 5-ASA to produce dense 5-ASA granules and/or agglomerates is also known (see for example, WO2013/144176). However, known high-shear wet granulation of 5-ASA generally requires the use of additional excipients such as fillers and lubricants.

Lactose is a commonly used filler or bulking agent for the granulation of 5-ASA due to its high water solubility, good technical properties (*e*.*g*. compactibility and flowability) and relatively low price. However, the interaction between reducing sugars such as lactose and 5-ASA has been related to the discoloration observed in certain commercially available 5-ASA products. This is thought to be due to the Maillard (or browning) reaction between the free amine group of 5-ASA and the "glycosidic" hydroxyl group of reducing sugars resulting in brown pigmented degradation products. In addition, the use of lactose in medicinal products is becoming less desirable due to the increasing prevalence of lactose intolerance. Lactose-free formulations of 5-ASA are disclosed in WO2004/087113, WO2009/047802 and US6773720.

In accordance with a first aspect of the present invention, there is provided a process for manufacturing 5-ASA tablet cores, the process comprising:
wet granulating a granulation composition comprising 5-ASA, a granulation liquid, and at least one binder using a high-shear granulator to form 5-ASA granules;
drying the 5-ASA granules to form dry granules;
blending the dry granules with at least one filler and optionally one or more additional pharmaceutical excipients to form a compression blend; and
compressing the compression blend to form 5-ASA tablet cores;
wherein the granulation composition and the compression blend do not comprise a reducing sugar.

A reducing sugar is a sugar that is capable of acting as a reducing agent because it has a free aldehyde or ketone group. Examples of reducing sugars include lactose, maltose, glucose and fructose.

Typically, the process of the present invention advantageously produces 5-ASA granules having sufficiently high density, good flowability and uniform particle size without the need for fillers in the granulation step. In addition, the preparation of 5-ASA granules using a high-shear granulator can be performed in a few minutes using a single piece of equipment. In contrast, in order to obtain 5-ASA granules having acceptable density when using a fluid bed granulation process, a dry pre-compaction step is usually require. The pre-compaction step is typically carried out in a dedicated separate piece of equipment. Moreover, scale-up of high-shear granulation processes is typically easier than scale-up of fluid bed granulation processes.

The quality of the 5-ASA granules produced according to the wet high-shear granulation process of the present invention allows the tableting process to be predictable and will produce quality tablets. The 5-ASA granules produced according to the present invention were successfully compressed into tablet cores having a high and uniform 5-ASA content, whilst minimizing the level of excipients in the tablet cores. In addition, the tablet cores according to the present invention maintain comparable critical attributes to commercially available 5-ASA tablets such as short disintegration time, low friability, and adequate hardness to enable coatings to be applied. In addition, the absence of reducing sugars such as lactose in the formulation prevents the undesirable Maillard reaction and also produces tablet cores that are suitable for patients intolerant to lactose.

The granulation liquid is typically a volatile solvent and may include, but is not limited to water, organic solvents such as ethanol, isopropyl alcohol, acetone, dichloromethane, or mixtures thereof. A preferred granulation liquid is water. The binder can be added as a solution with the granulation liquid or can be part of the powder bed which is then granulated with the granulation liquid. In preferred embodiments, the binder is dissolved in the granulation liquid to form a binder composition which is then added to the 5-ASA either all at once or, alternatively, slowly and continuously sprayed on to the 5-ASA bed (*e*.*g*. using a pneumatic or binary nozzle or via atomization using a pressure nozzle). Preferably, the granulation composition liquid consists of 5-ASA, a granulation liquid, and at least one binder (*i*.*e*. no other excipients are present in the granulation composition).

The binder can be any suitable binder known by the skilled person. Preferred synthetic polymer binders include, but are not limited to hydroxypropyl cellulose, hydroxypropyl methylcellulose (HPMC or hypromellose, *e*.*g*. Pharmacoat® 603), microcrystalline cellulose (*e*.*g*. Avicel® pH 102), methylcellulose, ethylcellulose, polyvinylpyrolidone (PVP), sodium carboxymethyl cellulose, polyvinylalcohol (PVA), polyethylene glycol 4000 (PEG 4000), and polyethylene glycol 6000 (PEG 6000). Preferred natural polymer binders include, but are not limited to starch, modified starch, pre-gelatinised starch, acacia gum, guar gum, tragacanth gum, xantham gum, gelatine, sucrose solution and maltodextrin solution. The binder is preferably present in an amount of about 6 wt % or less, preferably about 3 wt % or less, more preferably about 2 wt % or less, more preferably about 1 wt % or less, and most preferably about 0.5 wt % or less, based on the total weight of the tablet core.

High-shear wet granulation involves the agglomeration of powders through the addition of a granulation liquid and vigorous mixing. In high-shear granulation processes, powder densification occurs by applying a high-power-per-unit mass through mechanical forces. Typical high-shear granulators comprise a mixing bowl, a three-bladed impeller and a secondary impeller or chopper. In a typical high-shear granulation process, dry powders are mixed together by the impeller blade, which rotates through the powder bed. A liquid binder is then added and spread by the impeller and the chopper breaks down any wet, coarser agglomerates. Densification of the granules occurs during wet massing through impeller rotation. The tip speed of the impeller in typical high-shear granulators is from about 5 to about 15 m/s, or from about 6 to about 15 m/s, or from about 7 to about 12 m/s, or from about 8 to about 12 m/s, or from about 9 to about 11 m/s, e.g. in the order of about 10 m/s. In contrast, the tip speed of the impeller in a typical low-shear granulator is less than about 5 m/s.

The high-shear granulation step of the present invention can be carried out using any suitable high-shear granulator known in the art. Examples of suitable machines include a Dionsa high-shear mixer and a DEA high-shear mixer. The exact operating parameters of the high-shear granulator are dependent upon the system used. Details of conventional laboratory methods and techniques can be found in "Remington: The Science and Practice of Pharmacy 22nd Edition", published 3 Sept. 2012.

In the process of the present invention, the impeller of the high-shear granulator is typically operated at a rotation speed in the range of from about 500 rpm to about 700 rpm, more preferably from about 500 rpm to about 600 rpm, e.g. about 550 rpm. A secondary impeller or chopper can optionally be used to break down any wet, coarser agglomerates in the granules.

After granulation, the wet granules are preferably forced through a sieve to break up any large aggregates. The wet 5-ASA granules are then dried to produce dry granules having a residual moisture content (loss on drying) of less than about 1.5%, more preferably less than about 0.75%, more preferably less than about 0.5%, most preferably less than about 0.2%. Loss on drying (LOD) is determined according to the European Pharmacopoeia (Ph. Eur. 2.2.32). Preferably, the dry granules are screened, for example, through a low-shear mill to obtain a uniform and adequate particle size distribution (PSD).

It is preferred that the dry 5-ASA granules have a PSD in which the fraction of particles in the range of from 100 - 800 µm is from about 60% to 90%, preferably from about 65% to 85%, and most preferably from about 70% to 80%. PSD is measured according to the European Pharmacopoeia (Ph. Eur. 2.9.38).

It is preferred that the dry 5-ASA granules have a bulk density of at least about 500 g/l to about 800 g/l, preferably from about 550 g/l to about 750 g/l, more preferably from about 600 g/l to about 700 g/l. Bulk density is measured according to the European Pharmacopoeia (Ph. Eur. 2.9.34). Advantageously, the wet granulation step of the present invention produces 5-ASA granules having a bulk density 2 to 3 times greater than the original 5-ASA powder.

The dry 5-ASA granules are then blended with a filler and optionally one or more additional pharmacologically acceptable excipients to form a compression blend. Preferably, the excipients are pre-blended before blending with the granules. Optional pharmacologically acceptable excipients include, but are not limited to a disintegrant, *e*.*g*. sodium starch glycolate (*e*.*g*. Explotab® and Vivastar® P), a lubricant, e.g. magnesium stearate, and/or a flow regulator, *e.g.* colloidal silicon dioxide (*e*.*g*. Aerosil® 200).

The filler can be any suitable filler known by the skilled person, except for a reducing sugar. Preferred fillers include cellulosic fillers, *e*.*g*. cellulose, microcrystalline cellulose, ethyl cellulose and hydroxypropyl cellulose; sugar alcohols, *e*.*g*. erythritol, lactitol, mannitol, sorbitol and xylitol; non-reducing sugars, *e*.*g*. sucrose and trehalose; inorganic salts, *e*.*g*. dicalcium phosphate, calcium silicate, calcium sulfate, magnesium carbonate, sodium carbonate and sodium chloride; clays, *e*.*g*. talc and kaolin; and polysaccharides, e.g. dextrates, dextrin, maltodextrin, starch, modified starch and pre-gelatinised starch. The filler can be a single component or can be a mixture of components. It is particularly preferred that the filler is a cellulosic filler, preferably microcrystalline cellulose.

The filler is preferably present in an amount of about 20 wt % or less, preferably about 15 wt % or less, more preferably about 12 wt % or less, more preferably about 10 wt % or less, more preferably about 7.5% of less, for example about 5 wt %, based on the total weight of the tablet core.

The compression blend may optionally comprise one or more pharmacologically acceptable lubricants, *i.e.* an internal lubricant. Alternatively lubrication can be external. The lubricant is preferably present in the compression blend in an amount of about 2 wt % or less, preferably about 1 wt % or less, more preferably about 0.5 wt % or less based on the total weight of the coatable core.

The lubricant can be any suitable lubricant known by the skilled person. Preferred lubricants include by are not limited to. magnesium stearate, calcium stearate, stearic acid, hydrogenated vegetable oil (*e*.*g*. Sterotex®, Lubritab® and Cutina®), mineral oil, polyethylene glycol 4000-6000, sodium lauryl sulfate (SLS), glyceryl palmitostearate (*e*.*g*. Precirol®), glyceryl behenate (Compitrol® 888), sodium benzoate or sodium stearate fumarate. A particularly preferred lubricant is magnesium stearate.

It is preferred that the compression blend has a bulk density of at least about 600 g/l to about 800 g/l, preferably about 650 g/l to about 750 g/l. Preferably, the compression blend has a moisture content (LOD) in the range of from about 0.5% to about 2.0%, more preferably from about 0.7% to about 1.0%, and most preferably from about 0.7% to about 0.8%. Compression of the compression blend can be carried out using any suitable tableting machine known in the art. An example of a suitable tableting machine is an Erwerka eccentric single punch tablet press. The exact operating parameters for the tableting machine are dependent upon the system used.

Preferably, the compression speed during the compression step is from about 1,000 to about 70,000 tablets per hour, preferably from about 20,000 to about 65,000, more preferably from about 30,000 to about 60,000, more preferably from about 25,000 about 55,000 tablets per hour, *e*.*g*. about 50,000.

### The core

The "core" or "tablet core" is a solid body formed by compression on which a coating can be applied.

Preferably, the core has a hardness in the range of from about 80 N to about 200 N, or from about 100 N to about 190 N, or from about 120 N to about 180N, or from about 140 N to about 180 N, or from about 150 N to about 175 N, or from about 160 to about 170 N. Tablet core hardness is measured according to the European Pharmacopoeia (Ph. Eur. 2.9.8).

It is preferred that the core has a friability of 0% to less than about 0.5%, preferably less than about 0.25%, more preferably less than about 0.2%, most preferably from 0% to less than about 0.1%. Friability is defined as the tendency for a tablet or tablet core to chip, crumble or break following compression. Friability is measured according to the European Pharmacopoeia (Ph. Eur. 2.9.7).

It is preferred that the core has a disintegration time in water of less than about 15 minutes, preferably less than about 10 minutes as measured according to the European Pharmacopoeia (Ph. Eur. 2.9.1).

The core will typically comprise a therapeutically effective amount of 5-ASA, which may be from about 0.01 wt % to about 99 wt %, based on the total weight of the formulation. The actual dosage would be determined by the skilled person using his common general knowledge. However, by way of example, "low" dose formulations typically comprise no more than about 20 wt % of the 5-ASA, and preferably comprise from about 1 wt % to about 10 wt %, *e*.*g*. about 5 wt %, of 5-ASA. "High" dose formulations typically comprise at least 40 wt % of 5-ASA, and preferably from about 45 wt % to about 85 wt %, *e*.*g*. about 50 wt % or about 80 wt %.

Preferably, the 5-ASA tablet cores according to the present invention have a ratio of the total mass of the tablet core to the mass of 5-ASA of less than about 1.2, for example about 1.15.

The method according to present invention can be used to produce any size core containing a broad range of 5-ASA doses, for example, 5-ASA can be present in the core in an amount of from about 350 mg to about 1650 mg, or from about 450 mg to about 1650 mg, or from about 750 mg to about 1650 mg, or from about 1150 mg to about 1650 mg, or from about 1450 mg to about 1650 mg, or from about 1550 mg to about 1650 mg. Preferably, 5-ASA is present in the core in an amount selected from about 400 mg, about 800 mg, about 1200 mg, about 1500 mg, or about 1600 mg.

### Different Aspects

According to a further aspect of the present invention, there is provided a process for manufacturing a delayed release drug formulation for oral administration to deliver 5-ASA to the colon. The process comprises:
wet granulating a granulation composition comprising 5-ASA, a granulation liquid, and at least one binder using a high-shear granulator to form 5-ASA granules;
drying the 5-ASA granules to form dry granules;
blending the dry granules with at least one filler to form a compression blend;
compressing the compression blend to form 5-ASA tablet cores, and
coating the tablet cores with an outer coating preparation comprising a film-forming enteric polymer having a pH threshold at about pH 6 or above, and optionally, an enzymatically degradable polymer that is degraded by colonic enzymes to form coated tablet cores;
wherein the granulation composition and the compression blend do not comprise a reducing sugar.

Release from formulations according to the present invention is typically delayed until at least the distal ileum and, preferably, the colon. Release from certain formulations may also be sustained. However, in preferred formulations, release is pulsatile.

The outer coating preparation may be applied using the method described in WO2007/122374A.

The core can be uncoated or can pre-coated with either an isolation layer comprising a film-forming non-ionic polymer that is soluble in gastrointestinal fluid; or an inner layer comprising a polymeric material which is soluble in intestinal fluid or gastrointestinal fluid. Alternatively, the core can be pre-coated with both the isolation layer and the inner layer.

The time between initial exposure to conditions suitable for drug release and the start of drug release is known as the "lag time". The lag time depends on a number of factors including coating thickness and composition and may vary from one patient to the next. Formulations according to the present invention usually display a lag time in colonic conditions of at least 10 minutes. In most embodiments, the lag time is from about 10 minutes to about 8 hours. Complete release of the drug may be achieved in no more than 5 hours, *e*.*g*. no more than 4 hours, after exposure to these conditions.

A formulation is usually defined as gastric resistant if there is less than 10 wt % drug release in acidic media after 2 hours. Formulations according to the present invention typically display far less than 10 wt % drug release in acidic media and may be considered to be gastric resistant. The formulations usually display less than 1 wt % drug release in acidic media and, typically, display substantially no drug release in acidic media. When starch is combined with an acrylate film-forming material to form the outer layer of the coating for the core, typically less than 5 wt % drug release occurs over 5 hours in conditions simulating the stomach and small intestine.

### Film-forming enteric polymer (first polymeric material)

The film-forming enteric polymer (also referred to herein as the "first polymeric material) is pH sensitive and has a pH threshold at about pH 6 or above. The "pH threshold" is the pH below which it is insoluble in aqueous media, and at or above which it is soluble in aqueous media. The pH of the surrounding medium therefore triggers dissolution of the enteric polymer, and none (or essentially none) of the enteric polymer dissolves below the pH threshold. Once the pH of the surrounding medium reaches (or exceeds) the pH threshold, the enteric polymer becomes soluble.

By "insoluble" we mean that 1 g of a polymeric material requires more than 10,000 ml of solvent or "surrounding medium" to dissolve at a given pH.

By "soluble" we mean that 1 g of a polymeric material requires less than 10,000 ml, preferably less than 5,000 ml, more preferably less than 1000 ml, even more preferably less than 100 ml or 10 ml of solvent or surrounding medium to dissolve at a given pH.

"Surrounding medium", preferably means the medium in the gastrointestinal tract, such as gastric juice or intestinal juice. Alternatively, the surrounding medium may be the *in vitro* equivalent of the medium in the gastrointestinal tract.

The normal pH of gastric juice is usually in the range of pH 1 to 3. The enteric polymer is insoluble below pH 6, and soluble at about pH 6 or above and, thus, is usually insoluble in gastric juice.

The pH of intestinal fluid may vary from one person to the next, but in healthy humans is generally from about pH 5 to 6 in the duodenum, from about 6 to 8 in the jejunum, from about 7 to 8 in the ileum, and from about 6 to 8 in the colon. The enteric polymer preferably has a pH threshold of about 6.5, *i.e.* is insoluble below pH 6.5 and soluble at about pH 6.5 or above, and more preferably has a pH threshold of about 7 or above, *i.e.* is insoluble below pH 7 and soluble at about pH 7 or above. The enteric polymer typically has a pH threshold of no more than about pH 8, *e*.*g*. no more than about pH 7.5 and preferably no more than about pH 7.2. Preferably, the enteric polymer has a pH threshold within the range of pH found in the colon.

The pH threshold at which a polymer becomes soluble may be determined by a simple titration technique which would be part of the common general knowledge to the person skilled in the art.

Suitable enteric polymers contain groups that are ionisable in aqueous media to form anions. Such polymers are known in the art as "anionic" polymers. Suitable anionic polymers include polycarboxylic acid polymers, *i.e.* polymers or co-polymers that contain a plurality of carboxylic acid functional groups that are ionisable in aqueous media such as intestinal fluid, to form carboxylate anions. As the pH of the surrounding medium increases, the proportion of carboxylic acid groups that become carboxylate anions increases, thereby increasing the solubility of the polymer.

The film-forming enteric polymer is typically a polymethacrylate polymer, a cellulose polymer or a polyvinyl-based polymer. Examples of suitable cellulose polymers include cellulose acetate phthalate (CAP); cellulose acetate trimellitate (CAT); and hydroxypropylmethylcellulose acetate succinate (HPMC-AS). Examples of suitable polyvinyl-based polymers include polyvinyl acetate phthalate (PVAP).

Particularly suitable enteric polymers include a co-polymer of a (meth)acrylic acid and a (meth)acrylic acid C₁₋₄ alkyl ester, for example, a copolymer of methacrylic acid and methacrylic acid methyl ester. Such a polymer is known as a poly(methacrylic acid/methyl methacrylate) co-polymer. The ratio of carboxylic acid groups to methyl ester groups (the "acid:ester ratio") in these co-polymers determines the pH at which the co-polymer is soluble. The acid:ester ratio may be from about 2:1 to about 1:3, *e*.*g*. about 1:1 or, preferably, about 1:2. The molecular weight (MW) of preferred anionic co-polymers is usually from about 120,000 to 150,000 g/mol, preferably about 125,000 g/mol or about 135,000 g/mol.

Preferred anionic poly(methacrylic acid/methyl methacrylate) co-polymers have a molecular weight of about 125,000 g/mol. Suitable examples of such polymers have an acid:ester ratio of about 1:1 and a pH threshold of about pH 6, or have an acid:ester ratio of about 1:2 and a pH threshold of about pH 7.

A specific example of a suitable anionic poly(methacrylic acid/methyl methacrylate) co-polymer having a molecular weight of about 125,000 g/mol, an acid:ester ratio of about 1:1 and a pH threshold of about pH 6 is sold under the trade mark Eudragit® L. This polymer is available in the form of a powder (Eudragit® L 100), or as an organic solution (12.5%) (Eudragit® L 12.5).

A specific example of a suitable anionic poly(methacrylic acid/methyl methacrylate) co-polymer having a molecular weight of about 125,000 g/mol, an acid:ester ratio of about 1:2 and a pH threshold of about pH 7 is sold under the trade mark Eudragit® S. This polymer is available in the form of a powder (Eudragit® S 100) or as an organic solution (12.5%) (Eudragit® S 12.5).

The enteric polymer may be a co-polymer of methyl acrylate, methyl methacrylate and methacrylic acid. Preferred poly(methyl acrylate/methyl methacrylate/methacrylic acid) co-polymers have a molecular weight from about 250,000 to about 300,000 g/mol, e.g. about 280,000 g/mol. Suitable examples of such co-polymers have a methyl acrylate:methyl methacrylate:methacrylic acid ratio of about 7:3:1 thereby providing an acid:ester ratio of about 1:10 and a pH threshold of about pH 7.

A specific example of a suitable anionic poly(methyl acrylate/methyl methacrylate/ethyl acrylate) co-polymer is available in the form of an aqueous dispersion (30%) and is sold under the trade mark Eudragit® FS 30 D.

The Eudragit® co-polymers are manufactured and/or distributed by Evonik GmbH, Darmstadt, Germany.

Mixtures of film-forming enteric polymers may be used as appropriate. For example, the enteric polymer may actually be a blend of at least two different polymers having a pH threshold of about pH 6 or above. Preferably, the polymers in the blend are different polymethacrylate polymers. In embodiments where the enteric polymer is a blend of two different polymers having a pH threshold of about pH 6 or above, the polymers may be present in the blend in a polymer weight ratio from about 1:99 to about 99:1, *e*.*g*. from about 10:90 to about 90:10, or from 25:75 to about 75:25, or from about 40:60 to about 60:40, for example about 50:50.

An example of a suitable mixture would include a mixture, *e*.*g*. a 1:1 mixture, of Eudragit® L and Eudragit® S. A further example would include a blend, *e*.*g*. a 50:50 blend, of Eudragit® S and Eudragit® FS.

For the avoidance of doubt, the terms "mixture" and "blend" in the context of mixtures or blends of polymers forming the enteric polymer, are used herein interchangeably.

However, the use of a particular film-forming enteric polymer material, e.g. a poly(methacrylic acid/methyl methacrylate) co-polymer, alone is preferred. The use of Eudragit® S alone as the film-forming enteric polymer material is particularly preferred.

### Enzymatically degradable polymer (second polymeric material)

The enzymatically degradable polymer (also referred to herein as the "second polymeric material) is enzymatically degradable by one or more enzymes found in the colon of a subject. Such enzymes are produced by colonic bacteria and include amylases such as alpha-amylases, beta-amylases and iso-amlayses; amylopullunase, glucoamylase, alpha-glucosidase, maltogenic-amylase, glycosyltransferases and amylomaltase.

The person skilled in the art is capable of determining whether a material is susceptible to attack by colonic bacterial enzymes using techniques comprising part of the common general knowledge. For example, a pre-determined amount of a given material could be exposed to an assay containing an enzyme from a bacterium found in the colon and the change in weight of the material over time may be measured. Alternatively, the amount of a degradation product produced as a result of the action of the colonic bacterial enzymes could be measured.

The enzymatically degradable polymer is preferably a polysaccharide. Suitable polysaccharides are selected from the group consisting of starch; amylose; amylopectin; chitosan; chondroitin sulfate; cyclodextrin; dextran; pullulan; carrageenan; sclerglucan; chitin; curdulan and levan. The polysaccharide is preferably starch. Starches are usually extracted from natural sources such as cereals; pulses; and tubers. Suitable starches for use in the present invention are typically food grade starches and include rice starch; wheat starch; corn (or maize) starch; pea starch; potato starch; sweet potato starch; tapioca starch; sorghum starch; sago starch; and arrow root starch. The use of maize starch is exemplified below.

Starches are usually extracted from natural sources such as cereals; pulses; and tubers. Suitable starches for use in the present invention are typically food grade starches and include rice starch; wheat starch; corn (or maize) starch; pea starch; potato starch; sweet potato starch; tapioca starch; sorghum starch; sago starch; and arrow root starch. The use of maize starch is exemplified below.

Starch is typically a mixture of two different polysaccharides, namely amylose and amylopectin. Different starches may have different proportions of these two polysaccharides. Most natural (unmodified) maize starches have from about 20 wt % to about 30 wt % amylose with the remainder being at least substantially made up of amylopectin.

Suitable starches include "high amylose" and "low amylose" starches. High amylose starches are particularly preferred.

"High amylose" starches, are starches having at least 50 wt % amylose. Particularly suitable "high amylose" starches have from about 50 wt % to about 75 wt % amylose, preferably from about 50 wt % to about 70 wt %, more preferably from about 50 wt % to about 65 wt %, most preferably from about 50 wt % to about 60 wt %, *e*.*g*. about 55 wt %.

"Low amylose" starches are starches having less than 50 wt % amylose and at least 50 wt % amylopectin, *e*.*g*. up to 75 wt % amylopectin and even as much as up to 99 wt % amylopectin.

Starches suitable for use in the present invention typically have at least 0.1 wt %, *e*.*g*. at least 10 wt % or 15 wt %, preferably at least 35 wt %, amylose. Such starches have no more than 99.9 wt %, *e*.*g*. no more than 90 wt % or 85 wt %, preferably no more than 65 wt %, amylopectin. Such starches may have up to about 99 wt % amylose and no less than 1 wt % amylopectin.

Starches suitable for use in the present invention may have up to 100% amylopectin, more typically from about 0.1 wt % to about 99.9 wt % amylopectin. The starch may be, for instance, unmodified waxy corn starch. This typically comprises about 100% amylopectin.

Preferred starches have no more than 50 wt % amylopectin. Particularly suitable starches have from about 25 wt % to about 35 wt % amylopectin, *e*.*g*. about 30 wt % amylopectin.

The person skilled in the art is capable of determining the relative proportions of amylose and amylopectin in any given starch. For example, near-infrared (NIR) spectroscopy could be used to determine the amylose and amylopectin content of a starch using calibration curves obtained by NIR using laboratory-produced mixtures of known amounts of these two components. Further, starch could be hydrolysed to glucose using amyloglucosidase. A series of phosphorylation and oxidation reactions catalysed by enzymes result in the formation of reduced nicotinamide adenine dinucleotide phosphate (NADPH). The quantity of NADPH formed is stoichiometric with the original glucose content. Suitable test kits for this procedure are available (*e*.*g*., R-Biopharm GmbH, Germany). Another method that could be used involves subjecting the coating to digestion by bacterial enzymes, *e*.*g*. α-amylase, to produce short chain fatty acids (SCFA) which can be quantified by gas-liquid chromatography using a capillary column.

Preferred starches have amylose in its glassy form although amylose in its amorphous form may also be used in conjunction with the present invention.

Preferred starches are "off-the-shelf" starches, *i.e.* starches which require no processing prior to use in the context of the present invention. Examples of particularly suitable "high amylose" starches include Eurylon® 6 (or VI) or Amylo N-400 (Roquette, Lestrem, France), or Amylogel 03003 (Cargill, Minneapolis, USA) all of which are examples of a maize starch having from about 50 wt % to about 70 wt % amylose.

### Outer coating layer

In embodiments where the outer coating layer comprises a film-forming enteric polymer and an enzymatically degradable polymer, the proportion of the enzymatically degradable polymer to the film-forming enteric polymer is typically at least 1:99, *e*.*g*. at least 10:90 and preferably at least 25:75. The proportion is typically no more than 99:1, *e*.*g*. no more than 75:25 and preferably no more than 60:40. In some embodiments, the proportion may be no more than 35:65. In some preferred embodiments, the proportion is from 10:90 to 75:25, *e*.*g*. from 10:90 to 60:40 and preferably from 25:75 to 60:40. In some particularly preferred embodiments, the proportion is from 15:85 to 35:65, *e*.*g*. from 25:75 to 35:65 and preferably about 30:70. In other particularly preferred embodiments, the proportion is from 40:60 to about 60:40, *e*.*g*. about 50:50.

Optionally, conventional excipients such as those excipients selected from plasticisers for film formation (*e*.*g*. triethyl citrate), anti-tack agents (*e*.*g*. glyceryl monostearate (GMS) or talc) and surfactants (*e*.*g*. polysorbate 80), may be included in amounts up to 30 wt % of the final composition of the outer coating preparation.

The thickness of the outer coating of the core is typically from about 10 µm to about 300 µm. The thickness of a specific coating will, however, depend on the composition of the coating and the size of the core. For example, coating thickness is directly proportional to the amount of polysaccharide in the coating. Thus, in embodiments where the coating comprises high amylose starch and Eudragit® S at a ratio of about 30:70, the coating thickness may be from about 70 µm to about 130 µm, and preferably from about 90 µm to about 110 µm.

The outer coating typically has a coating amount of enteric polymer of from about 2 mg/cm² to about 10 mg/cm², *e*.*g*. from about 2 mg/cm² to about 8 mg/cm², or from about 3 mg/cm² to about 7 mg/cm², or from about 4 mg/cm² to about 6 mg/cm², or from about 5 mg/cm² to about 8 mg/cm², or from about 6 mg/cm² to about 8 mg/cm², or from about 7 mg/cm² to about 8 mg/cm², *e*.*g*. about 7.5 mg/cm², based on the dry weight of the enteric polymer. A typical core has a diameter of from about 5 x 10⁻⁴ m to about 25 mm.

The outer coating is preferably a single layer of a mixture of the polysaccharide and film-forming enteric polymer.

### Inner layer

The formulation according to the present invention optionally comprises an inner layer between the core and the outer later. The inner layer comprises a third polymeric material which is soluble in intestinal fluid or gastrointestinal fluid (both gastric and intestinal fluid).

By "gastric fluid", the inventors mean the aqueous fluid in the stomach of a mammal, particularly a human. The fluid contains up to about 0.1 N hydrochloric acid and substantial quantities of potassium chloride and sodium chloride, and plays a key role in digestion by activating digestive enzymes and denaturing ingested protein. Gastric acid is produced by cells lining the stomach and other cells produce bicarbonate which acts as a buffer to prevent the gastric fluid from becoming too acidic.

By "intestinal fluid", the Inventors mean the fluid in the lumen of the intestine of a mammal, particularly a human. Intestinal fluid is a pale yellow aqueous fluid secreted from glands lining the walls of the intestine. Intestinal fluid includes fluid found in the small intestine, *i.e.* fluid found in the duodenum (or "duodenal fluid"), fluid found in the jejunum (or "jejunal fluid") and fluid found in the ileum (or "ileal fluid"), and fluid found in the large intestine, *e*.*g*. "colonic fluid".

The skilled person can readily determine whether a polymer is soluble in gastric fluid and/or intestinal fluid. If a polymer is soluble in water (or aqueous solution, e.g. a buffer solution) at a pH from 1 to 3, then that polymer would typically be soluble in gastric fluid. Similarly if a polymer is soluble in water (or aqueous solution, *e*.*g*. a buffer solution) at a pH from 5 to 8, then that polymer would typically be soluble in intestinal fluid. Alternatively, the compositions of gastric fluid and intestinal fluid are known and may be replicated *in vitro.* If a polymer is soluble in artificial gastric fluid or intestinal fluid *in vitro,* then it would typically be soluble in gastric fluid or intestinal fluid respectively *in vivo.*

Any pharmacologically acceptable water soluble film-forming polymers are, in principle, suitable for use as the third polymeric material. The solubility of the water soluble polymers may be dependent on pH, *i.e.* the polymeric material may be a pH sensitive polymer having a pH threshold. In such embodiments, the pH threshold of the third polymeric material is less than, typically at least 0.5 pH units less than and preferably from 0.5 to 3.5 pH units less than, the pH threshold of the second polymeric material. The pH threshold of the third polymeric material is typically from about pH 4.5 to about pH 7.5.

The third polymeric material may be soluble in at least one fluid selected from gastric fluid, duodenal fluid, jejunal fluid and ileal fluid. However, in preferred embodiments, the solubility of the third polymeric material in water is not dependent on pH; at least not within the range of pH found in the intestine. In preferred embodiments, the third polymeric material is soluble in fluid at any point in the stomach and intestine, *i.e.* in gastrointestinal fluid.

Suitable polymers for use as the third polymeric material preferably contain groups that are ionisable in aqueous media to form anions. Such polymers are known in the art as "anionic" polymers. Suitable anionic polymers include polycarboxylic acid polymers, *i.e.* polymers or co-polymers that contain a plurality of carboxylic acid functional groups that are ionisable in aqueous media such as intestinal fluid, to form carboxylate anions.

In embodiments in which the third polymeric material is a polycarboxylic acid polymer, it is preferred that the third polymeric material is at least partially neutralised, *i.e.* that at least a portion, *e*.*g*. at least 10 %, preferably at least 25 %, more preferably at least 50 %, and most preferably at least 90 %, of the carboxylic acid groups are in the form of carboxylate anions. In particularly preferred embodiments, all of the carboxylic acid groups in the third polymeric material are in the form of carboxylate anions. Such polymers are referred to herein as "fully neutralised".

In preferred embodiments, the second and third polymeric materials are based on the same polycarboxylic acid polymer with the third polymeric material having a higher degree of neutralisation than the second polymeric material. For example, for a particular polycarboxylic acid polymer, the second polymeric material may be in non-neutralised form with the third polymeric material in partially or fully neutralised form. Alternatively, the second polymeric material may be in partially neutralised form, with the third polymeric material also in partially neutralised form (although partially neutralised to a greater extent), or in fully neutralised form.

Examples of suitable polycarboxylic acid polymers include cellulose acetate phthalate (CAP), polyvinyl acetate phthalate (PVAP), hydroxypropyl methylcellulose phthalate (HPMCP), hydroxypropyl methylcellulose acetate succinate (HPMC-AS), cellulose acetate trimellitate (CAT), xanthan gum, alginates and shellac. However, the polycarboxylic acid polymer is preferably selected from co-polymers of a (meth)acrylic acid and a (meth)acrylic acid alkyl, *e*.*g*. C₁₋₄ alkyl, ester and a copolymer of methacrylic acid and methacrylic acid methyl ester is particularly suitable. Such a polymer is known as a poly(methacrylic acid/methyl methacrylate) co-polymer or a "polymethacrylate". The ratio of carboxylic acid groups to methyl ester groups (the "acid:ester ratio") in these co-polymers determines the pH at which the co-polymer is soluble. The acid:ester ratio may be from about 2:1 to about 1:3, *e*.*g*. about 1:1 or, preferably, about 1:2. The molecular weight (MW) of preferred anionic co-polymers is usually from about 120,000 to 150,000, preferably about 125,000 or about 135,000.

Preferred co-polymers for the third polymeric material are discussed in detail in the section above relating to the second polymeric material, and include Eudragit® L; Eudragit® S; Eudragit® FS 30 D; Eudragit® L30D-55; and Eudragit® L100-55.

Preferably, the exemplary polymers are used as the third polymeric material is in at least partially, more preferably fully, neutralised form.

Partially neutralised polymers suitable for use as the third polymeric material, and their methods of production, are known in the art, for example from US2008/0200482A and WO2008/135090A. These polymers may be fully neutralised by the addition of further base to the coating solutions.

In preferred embodiments, the third polymeric material is an at least partially, preferably fully, neutralised co-polymer of (meth)acrylic acid and a (meth)acrylic acid C₁₋₄ alkyl ester. In particularly preferred embodiments, the third polymeric material is a fully neutralised co-polymer of (meth)acrylic acid and (meth)acrylic acid methyl ester, particularly Eudragit® S. The Inventors have observed that fully neutralised Eudragit® S is capable of forming a film and is readily and completely soluble in water independently of at least the range of pH found in the intestine, *e*.*g*. about pH 5 to about pH 8. Fully neutralised Eudragit® S is particularly preferred for use as the third polymeric material in the present invention.

Other polymers suitable for use as the third polymeric material include pharmacologically acceptable non-ionic polymers, *i.e.* pharmacologically acceptable polymers which do not ionise in aqueous media. In these embodiments, the inner layer additionally comprises at least one additive selected from a buffer agent and a base. In particular, the inner layer of these embodiments preferably comprises a base and, optionally, a buffer agent. In preferred embodiments, the inner layer comprises both a buffer agent and a base. Suitable examples of buffer agents and bases are discussed below.

Examples of suitable non-ionic polymers include methylcellulose (MC), hydroxypropyl cellulose (HPC), hydroxypropyl methylcellulose (HPMC), poly(ethyleneoxide)-graft-polyvinylalcohol, polyvinylpyrrolidinone (PVP), polyethylene glycol (PEG) and polyvinylalcohol (PVA).

Mixtures of film-forming polymer materials may be used as appropriate. The polymer components in such mixtures may be anionic polymers, non-ionic polymers, or a mixture of anionic and non-ionic polymers. An example of a suitable mixture would include a mixture, *e.g.* a 1:1 mixture, of Eudragit® L and Eudragit® S, and a mixture, *e.g.* a 1:1 mixture, of Eudragit® S and HPMC. However, the use of a particular film-forming polymeric material alone, *e*.*g*. a poly(methacrylic acid/methyl methacrylate) co-polymer and Eudragit® S in particular, is preferred.

In preferred embodiments, the inner layer comprises at least one base. The purpose of the base is to provide an alkaline environment on the underside of the outer layer once intestinal fluid begins to penetrate the outer layer. Without being bound by any particular theory, the Inventors believe that the alkaline environment facilitates dissolution and thereby also disintegration of the outer layer since the pH of the alkaline environment is above the pH threshold of the second polymeric material, thereby accelerating release of the drug from the formulation once the outer coating is dissolved and/or disintegrates.

In principle, any pharmacologically acceptable base may be used. The base is typically a non-polymeric compound. Suitable bases include inorganic bases such as sodium hydroxide, potassium hydroxide and ammonium hydroxide, and organic bases such as triethanolamine, sodium bicarbonate, potassium carbonate, trisodium phosphate, trisodium citrate or physiologically tolerated amines such as triethylamine.

The base is preferably selected from the group consisting of hydroxide bases, alkali metal bicarbonates, alkali metal carbonates, alkali metal phosphates, alkali metal citrates, or physiologically tolerated amines. More preferably, the base is a hydroxide base, and particularly preferred is sodium hydroxide.

In embodiments in which the third polymeric material is a fully neutralised polycarboxylic acid polymer, the base entrapped within the inner layer is usually the base that was used to neutralise the polymer and to adjust the pH of the inner coating preparation to a pH from about pH 7.5 to about pH 10 (see below).

In embodiments in which the third polymeric material is a non-ionic polymer, the inner layer usually comprises either a base, or more typically a combination of a base and a buffer agent.

The amount of base present in the inner layer would depend at least in part on the final pH of the inner coating preparation prior to coating a given batch of cores; the number of cores to be coated in the batch; the amount of the inner coating preparation used in the coating process of the batch.

The inner coating preferably comprises at least one buffer agent. The purpose of the buffer agent is to provide or increase buffer capacity on the underside of the outer layer once intestinal fluid begins to penetrate the outer layer. Without wishing to be bound by any particular theory, the Inventors believe that the buffer agent increases the buffer capacity in the dissolving inner layer and assists the ionisation and dissolution of the polymer in the outer layer; for a given pH, the higher the buffer capacity, the faster the rate of polymer dissolution. In embodiments where there is a base in the inner layer, the buffer agent helps maintains the alkaline environment under the outer layer once intestinal fluid penetrates the outer layer.

The buffer agent may be an organic acid such as a pharmacologically acceptable non-polymeric carboxylic acid, e.g. a carboxylic acid having from 1 to 16, preferably 1 to 3, carbon atoms. Suitable carboxylic acids are disclosed in WO2008/135090A. Citric acid is an example of such a carboxylic acid. The carboxylic acids may be used in carboxylate salt form, and mixtures of carboxylic acids, carboxylate salts or both may also be used.

The buffer agent may also be an inorganic salt such as an alkali metal salt, an alkali earth metal salt, an ammonium salt, and a soluble metal salt. As metals for the soluble metal salts, manganese, iron, copper, zinc and molybdenum can be mentioned. Further preferred, the inorganic salt is selected from chloride, fluoride, bromide, iodide, phosphate, nitrate, nitrite, sulphate and borate. Phosphates such as potassium dihydrogen phosphate are preferred over other inorganic buffer salts and organic acid buffers due to their greater buffer capacity at the pH of the coating solution, for example pH 8.

The buffer(s) is usually present in the inner layer in an amount from about 0.1 wt % to about 60 wt %, e.g. from about 0.1 wt % to about 50 wt %, preferably from about 0.1 wt % to about 40, more preferably from about 0.1 to about 20 wt %, more preferably from about 0.1 wt % to about 4 wt %, more preferably from about 0.1 wt % to about 3 wt %, and most preferably about 1 wt %, based on the dry weight of the third polymeric material.

In addition to the buffer agent and/or the base, the inner layer may comprise conventional excipients for polymer films, including those excipients selected from plasticizers (such a triethyl citrate), anti-tacking agents (such as GMS), and surfactants (such as polysorbate 80).

The thickness of the inner coating of the core is typically from about 10 µm to about 150 µm. The inner coating typically has a coating amount of polymer of from about 2 mg/cm² to about 10 mg/cm², preferably from about 2 mg/cm² to about 8 mg/cm², and most preferably from about 3 mg/cm² to about 7 mg/cm², based on the dry weight of the third polymeric material.

### Isolation layer

The formulation of the present invention may have an additional (or isolation) layer either between the active core and the inner layer and/or the outer layer.

There may be formulations according to the present invention in which the composition of the core is incompatible with the delayed release coating. In such cases, it may be desirable to include an isolation layer to separate the core from the coating. For example, the present invention embraces embodiments in which the inner layer provides an alkaline environment which is thought to assist in the dissolution and degradation of the outer layer. However, since 5-ASA has acidic groups, the inner layer may be incompatible with the core. In such cases, it would typically be appropriate to include an isolation layer.

Any suitable isolation layer known to the skilled person can be used. In one preferred embodiment, the isolation layer comprises a non-ionic polymer. Suitable non-ionic polymers include methylcellulose (MC); hydroxypropyl cellulose (HPC); hydroxypropyl methylcellulose (HPMC); poly(ethyleneoxide)-graft-polyvinylalcohol; polyvinylpyrollidone (PVP); polyethylene glycol (PEG); and polyvinylalcohol (PVA). HPMC or PVA is preferred. Mixtures of non-ionic polymers may also be used. A preferred mixture is HPMC and PEG. The isolation layer can additionally comprise a plasticiser. Suitable plasticisers include but are not limited to polyethylene glycol, triethyl citrate (TEC), triacetin and acetyltriethyl citrate.

### Examples

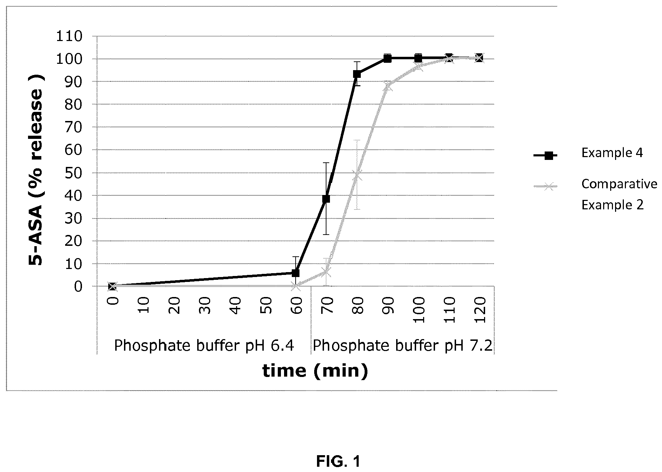

A number of preferred embodiments of the present invention will now be described with reference to the drawings, in which:-
FIG. 1 is a graph showing drug release as a function of time from (i) coated 5-ASA tablets according to Example 3 manufactured from 5-ASA granules produced by wet granulation in a high-shear mixer and (ii) commercially available 5-ASA tablets (Ref.) when exposed to 0.1M HCl for 2 hours and then to (a) phosphate buffer (pH 6.4) for 1 hour; and (b) phosphate buffer pH 7.2 for 1 additional hour.

### Preparation of 5-ASA Granules

### EXAMPLE 1 - Preparation of 5-ASA granules via wet granulation in a high-shear granulator

Mesalazine (5-ASA) was added to a high-shear mixer granulator (bowl size 500 g) and an aqueous composition of hydroxypropyl methylcellulose (Pharmacoat® 603) was added. Mixing was maintained at an impeller rotation speed of 500 rpm for about 20 minutes. The wet granules were passed through a Comil conical mill (6.4 mm square-holed screen) at a rotation speed of 2200 rpm before drying at a temperature of about 50°C until a loss on drying of less than 0.50% was achieved. The dry granules were screened through a Comil conical mill (1 mm square holed screen) at a rotation speed of about 2200 rpm.

The bulk density and sieve fraction of the granules produced according to Example 1 were measured and the results are summarised in Table 1 below along with the properties of granules produced via pre-compaction and wet granulation in a low-shear granulator (Comparative Example 1).

**Table 1**

| | **Example 1** | **Comparative Example 1** |
|---|---|---|
| | (High-shear granulator without filler) | (Dry compaction + wet granulation in fluid bed granulator including a filler) |
| Bulk density (g/mL) | 600-650 | 550-700 |
| Sieve fraction (%) (125-800 µm) | 70-80 | 70-80 |

Advantageously, the use of the one-step wet granulation process according to the present invention allows 5-ASA powder to be directly processed into granules without the need for an additional pre-compaction step. This has the advantage of reducing manufacturing costs and improving the efficiency of the manufacturing process.

Additional properties of the granules produced according to Example 1 were determined and are summarised in Table 2 below.

**Table 2**

| | **Example 1** |
|---|---|
| LOD (%) | 0.31 |
| Flow time (s) | 7 |
| Hausner-ratio | 1.20 |
| Angle of repose (°) | 35.3 |

### Preparation of 5-ASA tablet cores

### EXAMPLE 2 - Preparation of 800 mg lactose-free 5-ASA tablet cores (lab scale)

Oblong shaped 800 mg lactose-free 5-ASA tablet cores were prepared according to the following method. The amount of each component per tablet core is summarized in Table 1.

**Table 3**

| **Component** | **Amount per tablet core (mg)** |
|---|---|
| Mesalazine granules | 816 |
| Microcrystalline cellulose | 89 |
| Sodium starch glycolate | 27 |
| Colloidal silica | 1 |
| Magnesium stearate | 4.5 |
| **Total weight** | 937.5 |

Mesalazine (5-ASA) granules were prepared according to the method described in Example 1.

The dry mesalazine granules were blended for 10 minutes at 20 rpm with microcrystalline cellulose, (Avicel® PH 102), sodium starch glycolate (Explotab®) and talc in a cube blender. Magnesium stearate was then added and the resulting mixture was mixed for additional 5 minutes at 20 rpm.

Tableting was performed using a single punch eccentric tablet press equipped with a 17 x 8 mm tooling.

### EXAMPLE 3 - Preparation of 800 mg lactose-free 5-ASA tablet cores using PROSOLV SMCC 90 (lab scale)

Oblong shaped 800 mg lactose-free 5-ASA tablet cores were prepared according to the following method. The amount of each component per tablet core is summarized in Table 4.

**Table 4**

| **Component** | **Amount per tablet core (mg)** |
|---|---|
| Mesalazine granules | 816 |
| PROSOLV SMCC 90 | 71.5 |
| Sodium starch glycolate | 27.0 |
| Magnesium stearate | 5.5 |
| **Total weight** | 920 mg |

Mesalazine (5-ASA) granules were prepared according to the method described in Example 1.

PROSOLV SMCC 90 was blended with sodium starch glycolate (Explotab®). The resulting blend was added to the dry granules and blended in a cube blender for 10 minutes at 20 rpm. Magnesium stearate was then added and the resulting mixture was mixed for additional 5 minutes at 20 rpm.

Compression of the tableting blended was performed using an eccentric press single punch tableting machine equipped with a 17 x 8 mm tooling.

### Results

The physical properties of the 800 mg tablet cores produced in Examples 2 and 3 are summarised below in Table 5. Also provided are the properties for a commercially available 800 mg 5-ASA tablets comprising 5-ASA, microcrystalline cellulose and lactose.

**Table 5**

| | **Example 2** | **Example 3** | **Commercially available 5-ASA tablets** |
|---|---|---|---|
| Average tablet mass, n=20 (mg) | 937.9 ± 0.5% | 920.2 ± 0.5% | 1041.6 ± 0.7% |
| Average hardness, n=10 (N) | 177 | 162 | 172 |
| Average friability, n=10 (%) | 0.1 | 0.1 | 0.2 |
| Disintegration time in water, (min) | 2 | 3 | 6 |
| Thickness (mm) | 7.4 | 7.3 | 7.5 |

The data demonstrate that it is possible to consistently produce lactose-free 5-ASA tablets that have physical properties comparable to a commercially available 5-ASA tablets. Advantageously, the lactose-free tablet cores prepared according to the process of the present invention have a 10% lower tablet mass than commercially available 5-ASA products.

The elimination of lactose from the tablet cores removes the possibility for the undesirable Maillard reaction to occur. In addition, the lactose-free tablet cores of the present invention are suitable for patients who suffer from lactose intolerance.

### Preparation of coated tablet cores

### EXAMPLE 4 - Preparation of Eudragit® S 100 coated 800 mg 5-ASA tablet cores (laboratory scale)

The tablet cores of Example 3 were coated with a single outer layer of methacrylic acid-methyl methacrylate copolymer, ratio 1:2 (Eudragit® S 100).

The outer coating was applied from an alcoholic Eudragit® S 100 solution in the following amounts (Table 6).

**Table 6**

| **Component** | **Amount per tablet (mg)** |
|---|---|
| Eudragit® S 100 | 33.3 |
| Triethyl citrate | 6.64 |
| Talc | 8.92 |
| Pigments | 5.15 |

The alcoholic Eudragit® S 100 solution was prepared by dissolving Eudragit® S 100 into a solution of triethyl citrate in isopropyl alcohol and distilled water. The mixture was stirred for 35 minutes at room temperature.

A pigment suspension was added to the alcoholic Eudragit® S 100 solution and the mixture stirred for 10 minutes. A solution of triethyl citrate and isopropyl alcohol was added and the resulting outer layer coating preparation was stirred at room temperature for a further 15 minutes.

The pigment suspension was prepared by suspending talc (micronized) and the pigments in isopropyl alcohol. The suspension was homogenized for approximately 5 minutes.

The outer layer coating preparation was sprayed onto the to the 5-ASA tablet cores using a pan coater until the a coating amount of 7.5 mg/cm² Eudragit® S 100 was obtained.

### Drug release test #1 - Acid resistance studies followed by simulated fasted state

Acid resistance studies were performed on a USP type II apparatus using a paddle speed of 50 rpm and a media temperature of 37 ± 0.5 °C. To simulate the fasted state, tablets were tested in 0.1 M HCI for 2 hours.

### Drug release test #2 - Simulated fasted state then dissolution in phosphate buffer at pH 6.4 (1 hour) followed by phosphate buffer pH 7.2 (2 hours)

*In vitro* dissolution studies were performed on a USP type II apparatus using a paddle speed of 50 rpm and a media temperature of 37 ± 0.5 °C. To simulate the fasted state, tablets were first tested in 0.1 M HCI for 2 hours followed by 1 hour in phosphate buffer pH 6.4 and subsequently 1 hour in phosphate buffer pH 7.2.

### Results

No drug release was detected for coated tablets of Example 3 after 2 hours exposure to simulated fasted state (drug release test #1).

The data in FIG.1 show that the drug release profile for the coated 5-ASA tablets of Example 4 is comparable to that of commercially available coated tablets comprising 5-ASA, microcrystalline cellulose and lactose. Specifically, both tablets were resistant in pH 6.4 buffer (drug release of <10%) demonstrating the robustness of the coated tablets during transit through the small intestine (drug release test #2). In aqueous solution at pH 7.2, in both cases, rapid drug release was observed. Notably, the lactose-free formulations of Example 4 show even faster drug release than the commercially available coated 5-ASA tablets (Comparative Example 2).

It will be appreciated that the invention is not restricted to the details described above with reference to the preferred embodiments but that numerous modifications and variations can be made without departing from the scope of the invention as defined by the following claims.

## Claims

**1.** A process for manufacturing 5-aminosalicylic acid (5-ASA) tablet cores, said process comprising:
wet granulating a granulation composition comprising 5-ASA, a granulation liquid,
and at least one binder using a high-shear granulator to form 5-ASA granules;
drying said 5-ASA granules to form dry granules;
blending said dry granules with at least one filler and optionally one or more additional pharmaceutical excipients to form a compression blend; and
compressing said compression blend to form 5-ASA tablet cores;
wherein said granulation composition and said compression blend do not comprise a reducing sugar.

**2.** A process according to Claim 1, wherein said at least one filler is selected from the group consisting of dicalcium phosphate, calcium silicate, calcium sulfate, cellulose, microcrystalline cellulose, dextrates, dextrin, erythritol, ethyl cellulose, hydroxypropyl cellulose, kaolin, lactitol, magnesium carbonate, maltitol, maltodextrin, mannitol, sodium carbonate, sodium chloride, sorbitol, starch, modified starch, pre-gelatinised starch, sucrose, talc, trehalose and xylitol.

**3.** A process according to Claim 1 or Claim 2, wherein said filler comprises a cellulosic filler.

**4.** A process according to any of the preceding claims, wherein said filler comprises microcrystalline cellulose.

**5.** A process according to any of the preceding claims, wherein said filler is present in an amount of about 20 wt % or less, preferably about 10 wt % or less, more preferably about 5 wt % or less, based on the total weight of the tablet core.

**6.** A process for preparing 5-ASA granules, said process comprising wet granulating a composition consisting of 5-ASA, a granulation liquid, and at least one binder using a high-shear granulator to form 5-ASA granules.

**7.** A process according to any of the preceding claims, wherein said granulation liquid and said binder are mixed together to form a binder composition prior to adding to the 5-ASA.

**8.** A process according to any of the preceding claims, wherein said granulation liquid comprises water.

**9.** A process according to Claim 8, wherein said granulation liquid is water.

**10.** A process according to any of the preceding claims, wherein said binder comprises a cellulosic binder.

**11.** A process according to Claim 10, wherein said binder comprises hydroxypropyl methylcellulose (HPMC).

**12.** A process according to any of the preceding claims, wherein said binder is present in an amount of about 6 wt % or less, or about 3 wt % or less, or about 2 wt % or less, based on the total weight of the tablet core.

**13.** A process according to any of the preceding claims, wherein said one or more additional pharmacologically acceptable excipients comprises a disintegrant.

**14.** A process according to Claim 13, wherein said disintegrant comprises sodium starch glycolate.

**15.** A process according to any of the preceding claims, wherein said one or more additional pharmacologically acceptable excipients comprises a glidant.

**16.** A process according to Claim 15, wherein said glidant comprises colloidal silica.

**17.** A process according to any of the preceding claims, wherein said one or more additional pharmacologically acceptable excipients comprises a lubricant.

**18.** A process according to Claim 17, wherein said lubricant comprises magnesium stearate.

**19.** A process according to any of the preceding claims, wherein said dry granules have a bulk density of at least about 540 g/l.

**20.** A process according to any of the preceding claims, wherein said dry granules have a particle size distribution (PSD) in which the fraction of particles in the range of from 100 to 800 µm is from about 60% to 90%, preferably from about 65% to 85%, most preferably from about 70% to 80%.

**21.** A process according to any of the preceding claims, wherein 5-ASA is present in the core in an amount of from about 350 mg to about 1650 mg, or from about 450 mg to about 1650 mg, or from about 750 mg to about 1650 mg, or from about 1150 mg to about 1650 mg, or from about 1450 mg to about 1650 mg, or from about 1550 mg to about 1650 mg.

**22.** A process according to Claim 21, wherein 5-ASA is present in the core in an amount selected from about 400 mg, about 800 mg, about 1200 mg, about 1500 mg, or about 1600 mg.

**24.** A process according to any of the preceding claims, wherein the tablet core has a friability of less than about 0.5%, preferably less than about 0.4%, more preferably less than about 0.3%, more preferably less than about 0.2%, most preferably less than about 0.1%.

**25.** A process according to any of the preceding claims, wherein the tablet core has a disintegration time in water of less than about 15 minutes, preferably less than about 10 minutes, most preferably less than about 5 minutes.

**26.** A 5-ASA tablet core wherein the core does not comprise a reducing sugar, and wherein the ratio of the total mass of the tablet core to the mass of 5-ASA in the tablet core is less than 1.2.
